(19) Europäisches Patentamt
European Patent Office
Office européen des brevets

(11) Veröffentlichungsnummer: **0 031 591**
**B1**

(12) # EUROPÄISCHE PATENTSCHRIFT

(45) Veröffentlichungstag der Patentschrift: **21.09.83**

(51) Int. Cl.³: **C 07 J 43/00**, A 61 K 31/58

(21) Anmeldenummer: **80108189.4**

(22) Anmeldetag: **29.12.80**

(54) **2,2-Dimethyl-3-oxoandrosta-4,6-dien-17S-spiro-5'-(2'-oxo-3'-methyloxazolidin), Verfahren zu seiner Herstellung und dieses enthaltende Arzneimittel.**

(30) Priorität: **28.12.79 HU GO001463**

(43) Veröffentlichungstag der Anmeldung: **08.07.81 Patentblatt 81/27**

(45) Bekanntmachung des Hinweises auf die Patenterteilung: **21.09.83 Patentblatt 83/38**

(84) Benannte Vertragsstaaten: **BE DE FR GB IT NL**

(56) Entgegenhaltungen:
**FR-A-2 383 970**
**GB-A-2 017 708**
**US-A-3 272 801**

(73) Patentinhaber: **RICHTER GEDEON VEGYESZETI GYAR R.T., Gyömröi ut 19-21, H-1475 Budapest X (HU)**

(72) Erfinder: **Szilágyi, Katalin geb. Farago, Dr., Szakasits A. ut 60/a, Budapest XI. (HU)**
Erfinder: **Solyom, Sándor, Dr., Füredi park 7, Budapest XIV. (HU)**
Erfinder: **Toldy, Lajos, Dr., Villányi ut 53, Budapest XI. (HU)**
Erfinder: **Schäfer, Inge, Dr., Gárdos Mariska u. 6, Budapest III. (HU)**
Erfinder: **Szondy, Eleonora, Ujvilág u. 27, H-1145 Budapest (HU)**
Erfinder: **Borvendég, János, Dr., Széher u. 19, Budapest II (HU)**
Erfinder: **Hermann, Ilona geb. Szente, Ürömi ut 52, Budapest II. (HU)**

(74) Vertreter: **Beszédes, Stephan G., Dr., Am Heideweg 2 Postfach 1168, D-8060 Dachau (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

2,2-Dimethyl-3-oxoandrosta-4,6-dien-17S-spiro-5'-(2'-oxo-3'-methyloxazolidin), Verfahren zu seiner Herstellung und dieses enthaltende Arzneimittel

Die Erfindung betrifft das neue substituierte Steroid-17S-spirooxazolidinonderivat 2,2-Dimethyl-3-oxoandrosta-4,6-dien-17S-spiro-5'-(2'-oxo-3'-methyloxazolidin), ein Verfahren zu seiner Herstellung und diese Verbindung enthaltende Arzneimittel, insbesondere solche mit Antialdosteronwirkung bzw. antimineralcorticoider Wirkung, also Diuretica.

Es ist bekannt, dass das Aldosteron, das Hormon der Nebennierenrinde, eine Natriumretention verursacht und die Kaliumausscheidung erhöht. In krankhaften Fällen wird mehr Aldosteron erzeugt, was zur Bildung von Ödemen infolge einer gestörten Leber- und Nierentätig O ›wie Ödemen carialen Ursprunges führt. In diesen Fällen ist im Blut immer ein hoher Aldosteronspiegel nachweisbar.

Bei den genannten Krankheitsbildern wird die schädliche Wirkung des Hormones durch Verbindungen mit Aldosteron entgegenwirkender bzw. aldosteron-antagonistischer Wirkung gehemmt. Diese fördern über die Kanälchenzellen bzw. Tubuluszellen der Niere die Ausscheidung der Natriumionen und damit die Entleerung der Ödeme. Die Verbindungen mit Aldosteron entgegenwirkender Wirkung üben auf diese Weise eine diuretische Wirkung aus und bilden eine besonders bedeutende Gruppe der Diuretica. Sie werden unter anderem zur Behandlung von hohem arteriellem Blutdruck und Herzschwäche verwendet.

Ferner sind aus der bekanntgemachten britischen Patentanmeldung 2 017 708 Steroidspirooxazolidinonderivate der allgemeinen Formel

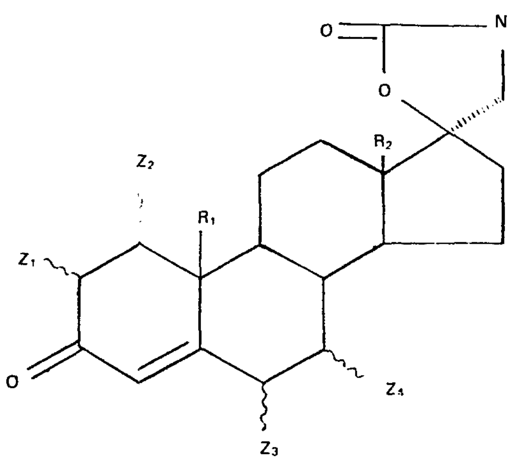

O

worin

$R_1$ für Wasserstoff oder einen Methylrest steht,

$R_2$ einen $C_{1-4}$-Alkylrest bedeutet,

$R_3$ Wasserstoff, einen $C_{1-4}$-Alkylrest oder einen $C_{2-4}$-Alkenylrest darstellt,

$Z_1$ und $Z_2$ jeweils für Wasserstoff stehen oder zusammen eine zweite Valenzbindung darstellen oder

$Z_1$ für Wasserstoff steht und

$Z_2$ einen Rest der Formel $R_4$ - S - bedeutet,

$Z_3$ und $Z_4$ jeweils für Wasserstoff stehen oder zusammen eine zweite Valenzbildung darstellen oder

$Z_3$ für Wasserstoff steht und

$Z_4$ einen Rest der Formel $R_4$ - S - bedeutet,

jedoch mindestens 1 der Paare $Z_1$ - $Z_2$ und $Z_3$ - $Z_4$ für Wasserstoff und einen Rest der Formel $R_4$ - S - steht, und

$R_4$ für Wasserstoff oder einen Alkyl-, Alkenyl-, Aralkyl- bzw. Acylrest mit bis zu 7 Kohlenstoffatomen steht,

mit der weiteren Massgabe, dass,

falls $Z_1$ und $Z_2$ zusammen für eine Valenzbindung stehen oder

$Z_1$ Wasserstoff bedeutet und

$Z_2$ einen Rest der Formel $R_4$ - S - darstellt,

$R_1$ nur für einen Methylrest stehen kann,

und ihre Stereoisomere sowie ihre Verwendung als diuretische Mittel vom Antialdosterontyp bekannt.

Weiterhin sind aus der französischen Offenlegungsschrift 2 383 970 Steroidspirooxazolidine der allgemeinen Formel

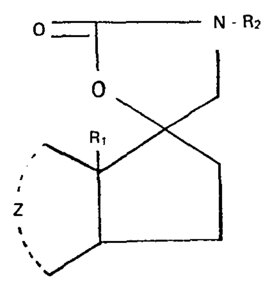

Oa

worin

$R_1$ für einen Alkylrest mit 1 bis 4 Kohlenstoffatomen steht,

$R_2$ Wasserstoff, einen Alkylrest mit 1 bis 4 Kohlenstoffatomen, einen Alkenylrest mit 1 bis 4 Kohlenstoffatomen oder einen Dialkylphosphinoxymethylrest mit einem Alkylteil mit 1 bis 3 Kohlenstoffatomen bedeutet und

Z unter anderen Reste der Formeln

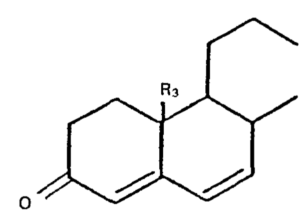

bzw.

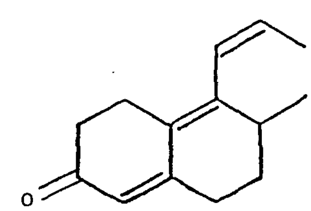

darstellen,

und ihre Stereoisomere sowie ihre diuretische Wirkung vom Antialdosterontyp bekannt.

Ausserdem sind in der US-Patentschrift 3 272 801 Steroidspiroxazolidinone der allgemeinen Formel

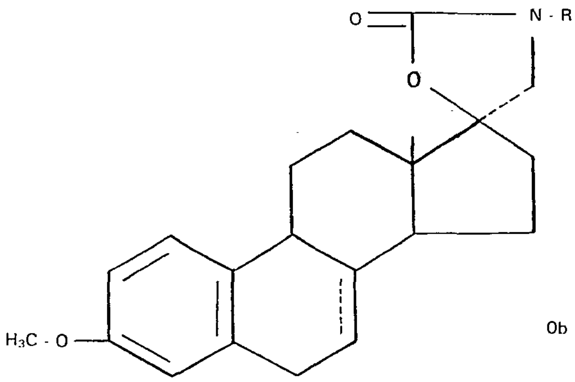

Ob

worin R für Wasserstoff oder einen niederen Alkylrest steht, und ihre antigonadotrophe Wirkung mit geringer östrogener Wirksamkeit und ihre Verwendung zur Schwangerschaftsregelung beschrieben.

Auch die in den letztgenannten 3 Druckschriften beschriebenen Verbindungen genügen nicht allen Ansprüchen voll.

Der Erfindung liegt die Aufgabe zugrunde, ein überlegene pharmakologische Wirkungen aufweisendes neues Steroid-17S-spirooxazolidinonderivat, ein Verfahren zu seiner Herstellung und diese Verbindung enthaltende Arzneimittel zu schaffen.

Das Obige wurde überraschenderweise durch die Erfindung erreicht.

Gegenstand der Erfindung ist 2,2-Dimethyl-3-oxo-androsta-4,6-dien-17S-spiro-5'-(2'-oxo-3'-methyl-oxazolidin) der Formel

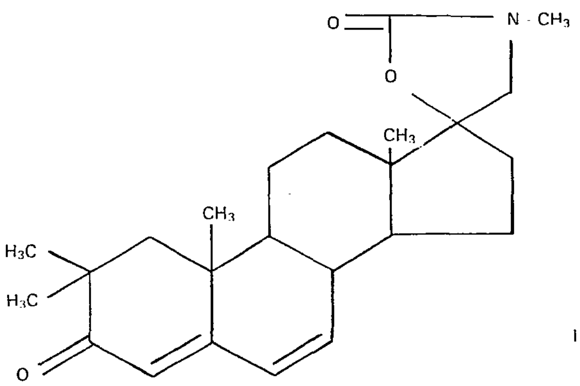

I

Von sämtlichen Verbindungen der bekanntgemachten britischen Patentanmeldung 2 017 708 unterscheidet sich die erfindungsgemässe Verbindung darin, dass in ihr in der 2-Stellung zwingend 2 Methylreste stehen, während nach der genannten Druckschrift dies nicht der Fall ist. Hinzukommt noch, dass die erfindungsgemässe Verbindung keine Alkylthio- oder Acylthiosubstituenten aufweist, während solche Substituenten für die Verbindungen der genannten Druckschrift in der bzw. den 2- und/oder 7-Stellung(en) zwingend festgelegt sind.

Die erfindungsgemässe Verbindung unterscheidet sich auch von sämtlichen Verbindungen der französischen Offenlegungsschrift 2 383 970 darin, dass in ihr im Gegensatz zu den letzteren in der 2-Stellung zwingend 2 Methylreste stehen.

Die erfindungsgemässe Verbindung liegt noch weiter von den Verbindungen der US-Patentschrift 3 272 801 entfernt. Zusätzlich zu den obigen Unterschieden kommt nämlich hinzu, dass in der erfindungsgemässen Verbindung in der 3-Stellung zwingend eine Oxogruppe vorliegt, während in dieser Stellung in den Verbindungen der genannten Druckschrift ein Methoxyrest steht, sowie in der erfindungsgemässen Verbindung zwischen den Kohlenstoffatomen in den 4- und 5-Stellungen und zwischen den Kohlenstoffatomen in den 6- und 7-Stellungen zwingend Doppelbindungen vorliegen, während in den Verbindungen der genannten Druckschrift die Doppelbindungen an ganz anderer Stelle liegen, nämlich zwischen den Kohlenstoffatomen in den 1- und 2-Stellungen, in den 3- und 4-Stellungen und 5- und 10-Stellungen sowie gegebenenfalls 7- und 8-Stellungen.

Gegenstand der Erfindung ist auch ein Verfahren zur Herstellung der erfindungsgemässen Verbindung, welches dadurch gekennzeichnet ist, dass in an sich bekannter Weise substituierte 3-Oxosteroid-17S-spirooxazolidinone der allgemeinen Formel

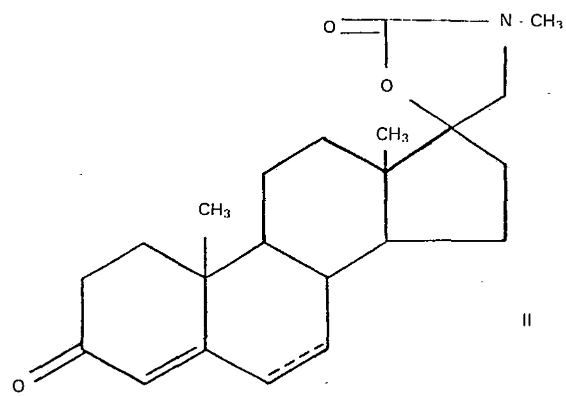

II

worin

die gestrichelte

Linie (----) für eine gegebenenfalls vorliegende weitere chemische Bindung steht,

in Gegenwart von Basen mit Methylhalogeniden methyliert werden und in den erhaltenen substituierten Steroid-17S-spirooxazolidinonderivaten, sofern deren Formel sich von der Formel I im Fehlen von 1 weiteren Doppelbindung zwischen den Kohlenstoffatomen in den 6- und 7-Stellungen unterscheidet, in an sich bekannter Weise 1 weitere Doppelbindung ausgebildet wird.

Vorzugsweise werden für die Methylierung der 3-Oxosteroid-17S-spirooxazolidinone der allgemeinen Formel II als Basen Alkalialkoholate, insbesondere Kalium-tert.-butylat, verwendet.

Es ist auch bevorzugt, für die Methylierung der 3-Oxosteroid-17S-spirooxazolidinone der allgemeinen Formel II als Methylhalogenid Methyljodid zu verwenden.

Zweckmässig wird die Methylierung der 3-Oxosteroid-17S-spirooxazolidinone der allgemeinen Formel II bei Raumtemperatur oder in der Kälte, vorzugsweise bei Temperaturen von −100 bis +30°C, durchgeführt.

Nach einer Ausführungsform des erfindungsgemässen Verfahrens werden die $\alpha,\beta$-ungesättigten 3-Oxosteroid-17S-spirooxazolidinone der allgemeinen Formel II, die gegebenenfalls eine weitere konjugierte Doppelbindung aufweisen können, bei niedrigen Temperaturen, zweckmässig unter −30°C, methyliert, wobei die entsprechenden 2,2-Dimethylderivate der Formel I bzw. der Formel, welche sich von der Formel I im Fehlen von 1 weiteren Doppelbindung zwischen den Kohlenstoffatomen in den 6- und 7-Stellungen unterscheidet, oder Gemische der entsprechenden 2,2-Dimethyl- und 2,2,4,4-Tetramethylderivate erhalten werden. Für diese Methylierung bei tiefen Temperaturen werden als Lösungsmittel vorzugsweise Äther, z.B. Tetrahydrofuran, verwendet.

Das bei der Methylierung erhaltene 2,2-Dimethyl-3-oxoandrosta-4,6-dien-17S-spiro-5'-(2'-oxo-3'-methyloxazolidin) der Formel I kann in an sich bekannter Weise, z.B. durch Eingiessen des Reaktionsgemisches in Wasser und anschliessendes Extrahieren, abgetrennt werden. Aus Gemischen mehrerer Derivate kann das erfindungsgemässe 2,2-Dimethyl-3-oxoandrosta-4,6-dien-17S-spiro-5'-(2'-oxo-3'-methyloxazolidin) unter anderem durch fraktionierte Kristallisation oder chromatographisch abgetrennt werden.

Das erfindungsgemäss gegebenenfalls erfolgende nachträgliche Einführen von 1 weiteren Doppelbindung wird am vor allem durch die Methylierung bei niedrigen Temperaturen erhaltenen 2,2-Dimethylderivat, dessen Formel sich von der Formel I im Fehlen von 1 weiteren Doppelbindung zwischen den Kohlenstoffatomen in den 6- und 7-Stellungen unterscheidet, vorgenommen.

So kann im erhaltenen 2,2-Dimethyl-3-oxosteroid-4-en-17S-spirooxazolidinon, dessen Formel sich von der Formel I im Fehlen von 1 weiteren Doppelbindung zwischen den Kohlenstoffatomen in den 6- und 7-Stellungen unterscheidet, die Doppelbindung zwischen den Kohlenstoffatomen in den 6- und 7-Stellungen z.B. durch Oxydation mit einem Benzochinonderivat ausgebildet werden. Als Benzochinonderivat werden zweckmässig Chloranil (Tetrachlor-p-chinon) oder Dichlordicyanbenzochinon verwendet.

Die 2,2-Dimethyl-3-oxosteroid-4-en-17S-spirooxazolidinonausgangsstoffe der allgemeinen Formel II können als solche oder in Form ihrer Enoläther eingesetzt werden.

Die als Ausgangsverbindungen verwendeten substituierten 3-Oxosteroid-17S-spirooxazolidinone der allgemeinen Formel II und ihre Herstellung sind in der belgischen Patentschrift 864 689 beschrieben.

Ferner sind erfindungsgemäss Arzneimittel, welche die erfindungsgemässe Verbindung als Wirkstoff, gegebenenfalls zusammen mit üblichen pharmazeutischen Konfektionierungsmitteln, enthalten, vorgesehen.

Die erfindungsgemässe Verbindung hat nämlich wie bereits erwähnt wertvolle pharmakologische Wirkungen, insbesondere Antialdosteronwirkung (dem Aldosteron entgegenwirkende bzw. aldosteron-antagonistische Wirkung) und somit diuretische Wirkung bei fehlenden hormonalen Wirkungen.

Die Antialdosteronwirkung der erfindungsgemässen Verbindung wurde mit Hilfe von 2 verschiedenen Verfahrensweisen untersucht:

a) Verfahrensweise nach C. M. Kagawa [C. M. Kagawa und Mitarbeiter: J. Pharmacology Exp. Ther., *126* (1959), 123].

18 Stunden vor der Behandlung wurden die Nebennieren der Versuchsratten entfernt. Gleichzeitig mit der Verarbreichung der zu untersuchenden Substanz erhielten die Tiere subkutan jeweils 12,5 µg Desoxycorticosteronacetat (DOCA), welches die Aldosteronwirkung ersetzt (Mineral-corticoid). Im Urin der Ratten wurde der $Na^+$- und $K^+$-Gehalt flammenphotometrisch bestimmt. Als Vergleichssubstanz wurde das anerkannt gut wirksame 17α-Carboxyäthyl-17β-hydroxy-7α-acetylthioandrost-4-en-3-onlacton [Spironolacton] gleicher Wirkungsrichtung wie die der erfindungsgemässen Verbindung in einer Menge von 480 µg/Tier verwendet. Zum Vergleich wurde auch eine Gruppe von Tieren nur mit Desoxycorticosteronacetat (DOCA) behandelt. Zur Auswertung wurden die Werte $\log \frac{Na^+ \times 10}{K^+}$ herangezogen. Die Ergebnisse sind in der unten folgenden Tabelle I zusammengestellt.

b) Verfahrensweise nach Holmann [Arch. Exp. Path. und Pharmak. *247* (1964), 419]; sogenanntes «Na-balance»-Experiment.

Es wurde ausgewählten männlichen Ratten mit Gewichten von 230 bis 250 g intravenös eine mit Glucose isoosmotisch gemachte 0,02%ige Natriumchloridinfusion verabreicht. Durch Beobachtung der $Na^+$-Ausscheidungsfähigkeit der Nieren während 24 Stunden wurde nachgewiesen, dass die mit dem Urin ausgeschiedene und die mit der Infusion in den Organismus eingebrachte $Na^+$-Menge in der Zeit zwischen der 4ten und der 13ten Stunde im Gleichgewicht waren. Es wurden 3 Gruppen gebildet:

1. Gruppe mit Mineral-corticoid + Antialdosteronverbindung.
2. Gruppe mit Mineral-corticoid.
3. Blindversuchsgruppe.

In der 2ten Gruppe wurden gleichzeitig mit dem Beginn der Infusion subkutan 6,25 µg Desoxycorticosteronacetat (DOCA)/Tier verabreicht. Durch diese einmalige Verabreichung des mineral-corticoiden Hormones der Nebennierenrinde wurde Hyperaldosteronismus hervorgerufen. Die Ratten der 1sten Gruppe erhielten neben den 6,25 µg Desoxycorticosteronacetat (DOCA)/Tier in der 2ten Stunde subkutan 5,5 mg erfindungsgemässe Verbindung bzw. 17α-Carboxyäthyl-17β-hydroxy-7α-acetylthioandrost-4-en-3-onlacton [Spironolacton]. Zur Auswertung wurden die im Urin messbare $Na^+$-Konzentration (ausgedrückt in $10^{-6}$ Mol/cm$^3$) und die je Stunde ausgeschiedene $Na^+$-Menge (ausgedrückt in $10^{-6}$ Mol/Stunde) herangezogen. Die Natriumretention wurde in Prozenten der eingebrachten $Na^+$-Menge ausgedrückt. Die Versuchsergebnisse sind in der unten folgenden Tabelle II zusammengestellt.

Tabelle I

Untersuchung der antimineral-corticoiden Wirkung bzw. Antialdosteronwirkung an Ratten nach Kagawa

| Beispiel | Untersuchte Verbindung Bezeichnung | Perorale Dosis in µg/Tier | Mineral-corticoid Bezeichnung | Subkutane Dosis in µg/Tier | Zahl der Tiere | Urin $\log \frac{Na^+ \times 10}{K^+}$ | Verhältnis des Wertes von $\log \frac{Na^+ \times 10}{K^+}$ zu dem von 17α-Carboxyäthyl-17β-hydroxy-7α-acetylthioandrost-4-en-3-onlacton [Spironolacton] |
|---|---|---|---|---|---|---|---|
| 1 | 2,2-Dimethyl-3-oxo-androsta-4,6-dien-17S-spiro-5'-(2'-oxo-3'-methyloxazolidin) | 480 | Desoxycorticosteronacetat (DOCA) | 12,5 | 8 | 1,66 | 1,37 |
| Vergleichssubstanz | 17α-Carboxyäthyl-17β-hydroxy-7α-acetylthioandrost-4-en-3-onlacton [Spironolacton] | 480 | Desoxycorticosteronacetat (DOCA) | 12,5 | 21 | 1,21 | 1 |
| Blindversuch | — | — | Desoxycorticosteronacetat (DOCA) | 12,5 | 28 | 0,78 | 0,65 |

Im folgenden wird zum Vergleich die entsprechende Tabelle der bekanntgemachten britischen Patentanmeldung 2 017 708 als Tabelle Ia gebracht. Die ihr zugehörigen Versuche wurden in derselben Weise wie die mit der erfindungsgemässen Verbindung, deren Ergebnisse in der obigen Tabelle I zusammengestellt sind, durchgeführt, nämlich nach derselben Verfahrensweise von C. M. Kagawa, wobei auch dieselbe perorale Dosis von 480µg/Tier verwendet wurde und dasselbe 17α-Carboxyäthyl-17β-hydroxy-7α-acetylthioandrost-4-en-3-onlacton [Spironolacton] als Vergleichssubstanz diente, so dass die beiden Versuche ohne weiteres miteinander vergleichbar sind.

Tabelle Ia

| Untersuchte Verbindung Bezeichnung | Perorale Dosis in ug/Tier | Urin $\log \frac{Na^+ \times 10}{K^+}$ | Verhältnis des Wertes von $\log \frac{Na^+ \times 10}{K^+}$ zu dem von 17α-Carboxyäthyl-17β-hydroxy-7α-acetylthioandrost-4-en-3-onlacton [Spironolacton] |
|---|---|---|---|
| 7α-(Acetylthio)-östr-4-en-3-on-17S-spiro-5'-(2'-oxo-3'-methyloxazolidin [Vergleichssubstanz A] {bekanntgemachte britische Patentanmeldung 2 017 708} | 480 | 1,43 | 0,94 |
| 17α-Carboxyäthyl-17β-hydroxy-7α-acetylthioandrost-4-en-3-onlacton [Spironolacton] {Vergleichssubstanz gegenüber der Vergleichssubstanz A} | 480 | 1,52 | 1 |
| Desoxycorticosteronacetat (DOCA) {Blindversuch} | — | 0,78 | 0,51 |

Tabelle Ia (Fortsetzung)

| Untersuchte Verbindung Bezeichnung | Perorale Dosis in ug/Tier | Urin $\log \frac{Na^+ \times 10}{K^+}$ | Verhältnis des Wertes von $\log \frac{Na^+ \times 10}{K^+}$ zu dem von 17α-Carboxyäthyl-17β-hydroxy-7α-acetylthioandrost-4-en-3-onlacton [Spironolacton] |
|---|---|---|---|
| 7α-(Äthylthio)-androst-4-en-3-on-17S-spiro-5'-(2'-oxo-3'-methyloxazolidin [Vergleichssubstanz B] {bekanntgemachte britische Patentanmeldung 2 017 708} | 480 | 1,09 | 1,04 |
| 17α-Carboxyäthyl-17β-hydroxy-7α-acetylthioandrost-4-en-3-onlacton [Spironolacton] {Vergleichssubstanz gegenüber der Vergleichssubstanz B} | 480 | 1,05 | 1 |
| Desoxycorticosteronacetat (DOCA) {Blindversuch} | – | 0,60 | 0,57 |

Tabelle II

Untersuchung der antimineral-corticoiden Wirkung bzw. Antialdosteronwirkung an Ratten nach Holmann

| Beispiel | Untersuchte Verbindung Bezeichnung | Subkutane Dosis in mg/Tier | Mineral-corticoid Bezeichnung | Subkutane Dosis in µg/Tier | Zahl der Tiere | Natriumretention in Gew.-% der eingebrachten Menge |
|---|---|---|---|---|---|---|
| 1 | 2,2-Dimethyl-3-oxaandrosta-4,6-dien-17S-spiro-5'-(2'-oxo-3'-methyloxazolidin) | 5,5 | Desoxycorticosteronacetat (DOCA) | 6,25 | 7 | 4,50 |
| Vergleichssubstanz | 17α-Carboxyäthyl-17β-hydroxy-7α-acetylthioandrost-4-en-3-onlacton [Spironolacton] | 5,5 | Desoxycorticosteron-acetat (DOCA) | 6,25 | 9 | 5,36 |
| Blindversuch | – | – | Desoxycorticosteronace-(DOCA) | 6,25 | 10 | 66,55 |
| Kontrollversuch | – | – | – | – | 6 | 22,68 |

Aus den obigen Tabellen I und II geht hervor, dass die antimineral-corticoide Wirkung bzw. Antialdosteronwirkung der erfindungsgemässen Verbindung wesentlich grösser als die der Vergleichssubstanz 17α-Carboxyäthyl-17β-hydroxy-7α-acetylthioandrost-4-en-3-onlacton [Spironolacton] ist. Darüberhinaus hat die erfindungsgemässe Verbindung gegenüber der Vergleichssubstanz den Vorteil, dass sie überraschenderweise keine Hormonwirkung zeigt.

Die bei der Behandlung des Hyperaldosteronismus bisher eingesetzten Verbindungen, z.B. das 17α-Carboxyäthyl-17β-hydroxy-7α-acetylthioandrost-4-en-3-onlacton [Spironolacton], haben nämlich den bedeutenden Nachteil, dass sie als Nebenwirkung bestimmte sexualspezifische Wirkungen, die bei längerer Therapiedauer früher oder später störend wirken, aufweisen. Besonders unerwünscht sind diejenigen Nebenwirkungen, welche auf die antiandrogene bzw. gestagene Wirkung der bekannten Antialdosteronmittel zurückzuführen sind.

Aus den obigen Tabellen I und Ia geht hervor, dass die erfindungsgemässe Verbindung eine 1,32mal

($\frac{1,37}{1,04}$) so hohe Wirkung bezüglich des Wertes von

$$\log \frac{Na^+ \times 10}{K^+}$$

im Urin wie die beste der untersuchten Verbindungen der bekanntgemachten britischen Patentanmeldung 2 017 708, nämlich 7α-(Äthylthio)-androst-4-en-3-on-17S-spiro-5'-(2'-oxo-3'-methyloxazolidin [Vergleichssubstanz B] hat.

Die antiandrogene Wirkung der erfindungsgemässen Verbindung der allgemeinen Formel I wurde mit Hilfe der modifizierten Dorfman-Verfahrensweise [R. I. Dorfman, D. F. Stevens: Endocrinology *67* (1960), 394] untersucht. Es wurden jugendliche bzw. kindliche kastrierte männliche Ratten mit Gewichten von 50 g 7 Tage lang täglich mit 50 µg Testosteronpropionat/Tier behandelt. Gleichzeitig wurde die zu untersuchende Substanz in einer Dosis von 1 mg/Tier verabreicht. Beide Substanzen wurden subkutan verabreicht. 8 Tage nach der Behandlung wurden die Tiere getötet und die Gewichte der ventralen Prostata und der Vesicula seminalis wurden nach ihrer Reinigung mittels einer Torsionswaage bestimmt. (Es ist bekannt, dass die Gewichtszunahme der sekundären Geschlechtsdrüsen durch antiandrogene Stoffe verringert wird. Das Mass der Hemmung kann bei Zugrundelegung der Gewichtszunahme der mit Testosteronpropionat angeregten bzw. stimulierten Drüsen als 100% in Gewichtsprozenten angegeben werden.).

Die Untersuchungen ergaben, dass die Vergleichssubstanz 17α-Carboxyäthyl-17β-hydroxy-7α-acetylthioandrost-4-en-3-onlacton [Spironolacton] bei ihrer Anwendung bei der 7tägigen Behandlung in Dosen von 0,5 und 1,0 $\frac{mg}{Tag \cdot Tier}$ die Gewichtszunahme der ventralen Prostata um 35 Gew.-% bzw. 45 Gew.-% und die Gewichtszunahme der Vesicula seminalis um 38 Gew.-% bzw. 54 Gew.-% hemmte. Demgegenüber zeigte die erfindungsgemässe Verbindung 2,2-Dimethyl-3-oxoandrosta-4,6-dien-17S-spiro-5'-(2'-oxo-3'-methyloxazolidin) in Dosen von 1 $\frac{mg}{Tag \cdot Tier}$ keine antiandrogene Wirkung.

Die gestagene Wirkung der erfindungsgemässen Verbindung der Formel I wurde nach der Verfahrensweise von C. Clauberg [C. Clauberg: Zentralblatt Gynaekol. *54* (1930) 2757] bestimmt. Es wurden jugendliche bzw. kindliche weibliche Neuseelandkaninchen 5 Tage lang täglich subkutan mit 5 µg Östradiol behandelt. In den folgenden Tagen wurden ebenfalls subkutan die zu untersuchenden Verbindungen verabreicht. Die beiden Hörnern der Gebärmutter bzw. des Uterus in 2 unterschiedlichen Höhen entnommenen Proben wurden nach einer histologischen Aufarbeitung gemäss McPhail ausgewertet.

Die Vergleichssubstanz 17α-Carboxyäthyl-17β-hydroxy-7α-acetylthioandrost-4-en-3-onlacton [Spironolacton] verursachte in einer Dosis von 5 × 1 mg/kg eine schwache gestagene Wirkung (Mc.Ph.-Index: 0,3) und in einer Dosis von 5 × 5 mg/kg bereits eine bedeutende gestagene Wirkung (Mc.Ph.-Index: 2,0). Die erfindungsgemässe Verbindung 2,2-Dimethyl-3-oxoandrosta-4,6-dien-17S-spiro-5'-(2'-oxo-3'-methyloxazolidin) erwies sich dagegen beim Versuch in den gleichen Dosen als inaktiv.

Die Erfindung wird anhand der folgenden Beispiele näher erläutert.

*Beispiel 1*

*2,2-Dimethyl-3-oxoandrosta-4,6-dien-17S-spiro-5'-(2'-oxo-3'-methyloxazolidin)*

Es wurden 4 g 3-Oxoandrost-4-en-17S-spiro-5'-(2'-oxo-3'-methyloxazolidin) in einem Gemisch aus 25 $cm^3$ Tetrahydrofuran und 12,5 $cm^3$ Methyljodid gelöst. Die Lösung wurde auf −60°C gekühlt. Dann wurde unter ständigem Rühren bzw. Schütteln in einer Stickstoffatmosphäre eine Suspension von 6 g Kalium-tert.-butylat in 30 $cm^3$ Tetrahydrofuran zugesetzt, wobei darauf zu achten war, dass die Temperatur nicht über −60°C stieg. Danach wurde das Reaktionsgemisch noch 1 Stunde bei −60°C gerührt bzw. geschüttelt und dann in 600 $cm^3$ Eiswasser eingegossen. Das Produkt wurde mit Dichlormethan extrahiert. Der Auszug wurde mit Wasser gewaschen, über Magnesiumsulfat getrocknet und dann eingedampft. Der Eindampfrückstand wurde aus Äthylacetat umkristallisiert. So wurden 2,34 g (54% der Theorie) 2,2-Dimethyl-3-oxoandrost-4-en-17S-spiro-5'-(2'-oxo-3'-methyloxazolidin) mit den folgenden Kenndaten erhalten.

Schmelzpunkt = 188 bis 190°C.

$[\alpha]_D^{20}$-Wert = +8,05° (c = 0,5, Chloroform).

UV-Spektrum: $_{max}^{C_2H_5OH}$ = 239 nm (E = 15000).

Verfahrensweise A)

4,76 g wie vorstehend beschrieben hergestelltes 2,2-Dimethyl-3-oxoandrost-4-en-17S-spiro-5'-(2'-oxo-3'-methyloxazolidin) wurden in 90 $cm^3$ Methanol gelöst. Zur Lösung wurden 3,16 g Chloranil und als Katalysator etwa 20 mg p-Toluolsulfonsäure zugegeben. Das Reaktionsgemisch wurde 15 Stunden lang zum Sieden erhitzt und dann unter verminderten Druck auf $^1/_3$ eingeengt. Das eingeengte Reaktionsgemisch wurde mit Wasser auf sein ursprüngliches Volumen aufgefüllt und dann 3mal mit Dichlormethan extrahiert. Die vereinigten organischen Phasen wurden mit einer 3 Gew.-% Natriumdithionit enthaltenden wässrigen n Natronlauge gewaschen, bis sich die Waschflüssigkeit nicht mehr verfärbte. Danach wurde die organische Phase mit Wasser gewaschen, über Magnesiumsulfat getrocknet und dann eingedampft. Es wurden 3,74 g Rohprodukt erhalten. Die aus Äthylacetat umkristallisierte in einer Menge von 2,1 g (44,4% der Theorie) erhaltene Substanz 2,2-Dimethyl-3-oxoandrosta-4,6-dien-17S-spiro-5'-(2'-oxo-3'-methyloxazolidin) hatte die folgenden Kenndaten.

Schmelzpunkt = 225 bis 228°C.

$[\ \ ]_D^{20}$-Wert = −31,8° (c = 0,5, Chloroform).

UV-Spektrum: $\lambda_{max}^{C_2H_5OH}$ = 281 nm (E = 23600).

Verfahrensweise B)

Es wurden 3,97 g 3-Oxoandrosta-4,6-dien-17S-spiro-5'-(2'-oxo-3'-methyloxazolidin) in einem Gemisch aus 12,5 $cm^3$ Methyljodid und 25 $cm^3$ Tetrahydrofuran gelöst. Die Lösung wurde auf −60°C gekühlt. Dann wurde unter ständigem Rühren bzw. Schütteln in einer Stickstoffatmosphäre eine Suspension von 6 g Kalium-tert.-butylat in 30 $cm^3$ Tetrahydrofuran zugesetzt, wobei darauf zu achten war, dass die Temperatur nicht über −60°C stieg. Danach wurde das Reaktionsgemisch noch 1 Stunde bei −60°C gerührt bzw. geschüttelt und dann in 600 $cm^3$ Eiswasser eingegossen. Das Produkt wurde mit Dichlormethan extrahiert. Der Auszug wurde mit Wasser gewaschen, über Magnesiumsulfat getrocknet und dann eingedampft. Das kristalline Rohprodukt wurde aus Äthylacetat umkristallisiert. So wurden als Produkt 2,72 g (63% der Theorie) 2,2-Dimethyl-3-oxoandrosta-4,6-dien-17S-spiro-5'-(2'-oxo-3'-methyloxazolidin), das mit dem gemäss der vorstehenden Verfahrenweise A) erhaltenen Produkt identisch war, erhalten.

*Beispiel 2*

Arzneimittelpräparat

Es wurden peroral verabreichbare Tabletten mit einem Wirkstoffgehalt von 25 mg der folgenden Zusammensetzung bereitet:

| | |
|---|---|
| 2,2-Dimethyl-3-oxoandrosta-4,6-dien-17S-spiro-5'-(2'-oxo-3'-methyloxazolidin) | 25 mg |
| Maisstärke | 128 mg |
| Polyäthylenglykol mit einem Molekulargewicht von 6000 | 40 mg |
| Talk | 6 mg |
| Magnesiumstearat | 1 mg |
| | 200 mg |

Die Tabletten wurden mit einem Film- oder Zuckerüberzug versehen.

**Patentansprüche**

1. 2,2-Dimethyl-3-oxoandrosta-4,6-dien-17S-spiro-5'-(2'-oxo-3'-methyloxazolidin) der Formel

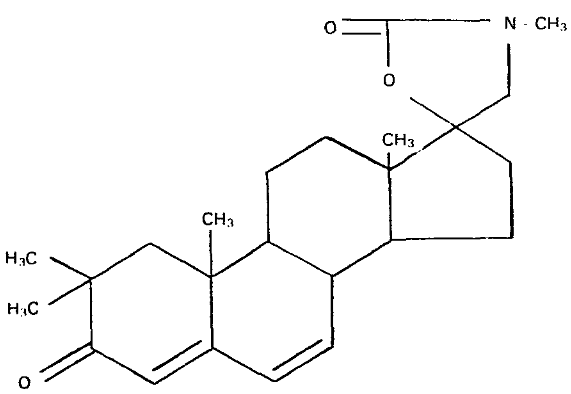

I

2. Verfahren zur Herstellung der Verbindung nach Anspruch 1, dadurch gekennzeichnet, dass man in an sich bekannter Weise substituierte 3-Oxosteroid-17S-spiro-oxazolidinone der allgemeinen Formel

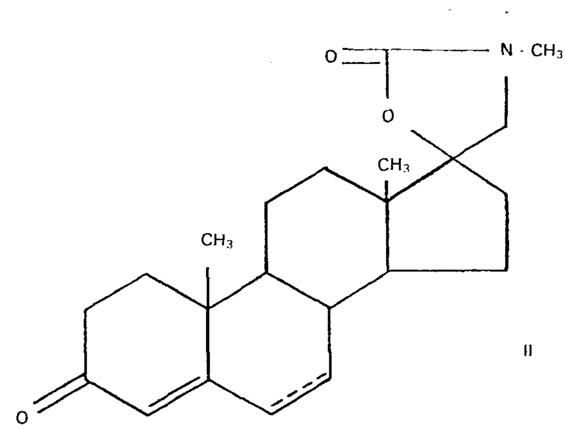

II

worin

die gestrichelte

Linie (----) für eine gegebenenfalls vorliegende weitere chemische Bindung steht,

in Gegenwart von Basen mit Methylhalogeniden methyliert und in den erhaltenen substituierten Steroid-17S-spirooxazolidinonderivaten, sofern deren Formel sich von der Formel I im Fehlen von 1 weiteren Doppelbindung zwischen den Kohlenstoffatomen in den 6- und 7-Stellungen unterscheidet, in an sich bekannter Weise 1 weitere Doppelbindung ausbildet.

3. Verfahren nach Anspruch 2, dadurch gekennzeichnet, dass man für die Methylierung der 3-Oxosteroid-17S-spirooxazolidinone der allgemeinen Formel II als Base Kalium-tert.-butylat verwendet.

4. Verfahren nach Anspruch 2 oder 3, dadurch gekennzeichnet, dass man für die Methylierung der 3-Oxosteroid-17S-spirooxazolidinone der allgemeinen Formel II als Methyhalogenid Methyljodid verwendet.

5. Verfahren nach Anspruch 2 bis 4, dadurch gekennzeichnet, dass man die Methylierung der 3-Oxosteroid-17S-spirooxazolidinone der allgemeinen Formel II bei Temperaturen von −100 bis +30°C durchführt.

6. Arzneimittel, gekennzeichnet durch einen Gehalt an der Verbindung nach Anspruch 1 als Wirkstoff, gegebenenfalls zusammen mit üblichen pharmazeutischen Konfektionierungsmitteln.

**Claims**

1. 2,2-dimethyl-3-oxoandrosta-4,6-dien-17S-spiro-5'-(2'-oxo-3'-methyloxazolidine) having the formula

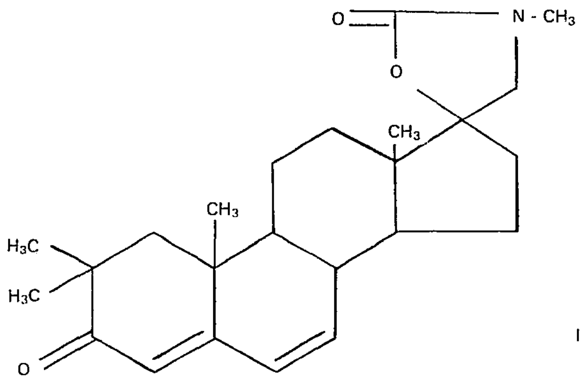

I

2. A process for preparing the compound according to claim 1, characterized in that in a manner known per se one methylates substituted 3-oxo-steroid-17S-spiro-oxazolidinones having the general formula

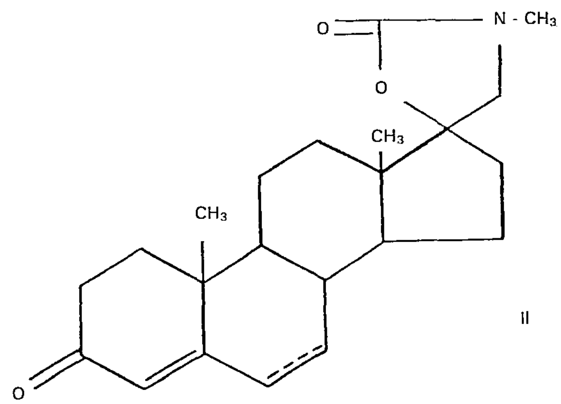

II

in which
the dotted line
(----) represents a further chemical bond optionally present
in the presence of bases with methyl halogenides and one develops in a manner known per se 1 further double bond in the obtained substituted steroid-17S-spirooxazolidinone derivatives in so far as the formula of them differs from the formula I in the lack of 1 further double bond between the carbon atoms in the 6- and 7-positions.

3. A process according to claim 2, characterized in that one uses for the methylation of the 3-oxo-steroid-17S-spirooxazolidinones of the general formula II as a base potassium tert.-butylate.

4. A process according to claim 2 or 3, characterized in that one uses for the methylation of the 3-oxosteroid-17S-spirooxazolidinones of the general formula II as a methyl halogenide methyl jodide.

5. A process according to claim 2 to 4, characterized in that one carries out the methylation of the 3-oxosteroid-17S-spirooxazolidinones of the general formula II at temperatures of from −100 to +30°C.

6. Medicaments, characterized by a content of a compound according to claim 1 as an active principle, optionally together with usual confectioning agents.

**Revendications**

1. 2,2-diméthyl-3-oxoandrosta-4,6-diène-17S-spiro-5'-(2'-oxo-3'-méthyloxazolidine) de formule

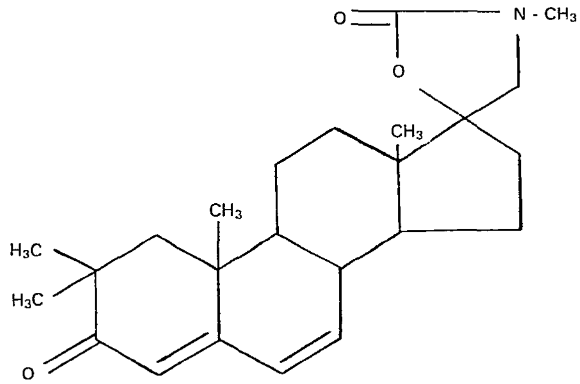

I

2. Procédé de préparation du composé selon la revendication 1 caractérisé en ce qu'on méthyle, d'une manière connue en elle-même, au moyen d'halogénures de méthyle, en présence de bases, 3-oxo-stéroïde-17S-spirooxazolidinones substituées de formule générale

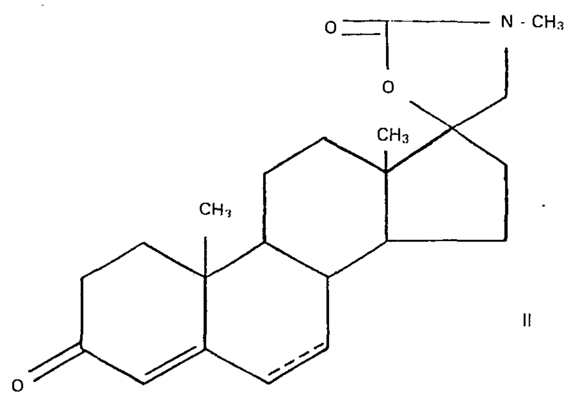

II

dans laquelle la ligne en traits discontinus (----) représente une liaison chimique supplémentaire existant éventuellement et en ce qu'on forme une double liaison supplémentaire, d'une manière connue en elle-même, dans les dérivés de stéroïde-17S-spirooxazolidinones substituées dans la mesure où leur formule se distingue de la formule I par l'absence d'une double-liaison supplémentaire entre les atomes de carbone qui se trouvent en positions 6 et 7.

3. Procédé selon la revendication 2, caractérisé en ce que pour la méthylation des 3-oxostéroïde-17S-spirooxazolidinones de formule générale II, on utilise comme base, le butylate tertiaire de potassium.

4. Procédé selon la revendication 2 ou 3, caractérisé en ce que pour la méthylation des 3-oxostéroïde-17S-spirooxazolidinones de formule générale II, on utilise comme halogénure de méthyle, l'iodure de méthyle.

5. Procédé selon la revendication 2 à 4, caractérisé en ce que l'on effectue la méthylation des 3-oxostéroïde-17S-spirooxazolidinones de formule générale II à des températures comprises entre −100 et +30°C.

6. Médicament caractérisé par une teneur en composé selon la revendication 1 comme substance active, éventuellement associé avec des agents de conditionnement pharmaceutiques usuels.